# EUROPEAN PATENT APPLICATION

(11) **EP 1 772 432 A1**
(43) Date of publication of application: **11.04.2007**
(21) Application number: 05719635.4
(22) Date of filing: 28.02.2005
(51) Int. Cl.: C02F 1/46

(54) **ACIDIC WATER PRODUCING DEVICE, ACIDIC WATER PRODUCING METHOD, AND ACIDIC WATER**

(30) Priority: 05.03.2004 JP 2004061678; 16.12.2004 JP 2004364963
(71) Applicant: Hosoda Electric Co., Ltd., Tokyo 144-0045 (JP)
(72) Inventor: HOSODA, Yuzo, c/o HOSODA ELECTRIC CO., LTD., Tokyo 1440045 (JP)
(74) Representative: Van Straaten, Joop
(86) International application number: PCT/JP2005/003307
(87) International publication number: WO 2005/085140

(57) **Abstract**

The present invention comprises:
-the electrolytic cell 2 where raw water is injected;
-diaphragm for electrolysis 3 which compart electrolytic cell to the acid water generation area 5 and the alkali water generation area 6;
-anode 24 placed in the acid water generation area 5;
-cathode 26 placed in the alkali water generation area 6;
-fossil earths put into the acid water generation area 5;
-voltage applying device which apply voltage to anode 24 and cathode 26;
-the filtering means 70 for filtering acid water generated at the acid water generation area 5; and
-heating means 80 for heating acid water filtered by the filtering means 70.

## Description

### Field of the Invention

The invention relates to an acid water generation device, a method for generating acid water, and acid water, particularly an acid water generation device, a method for generating acid water and acid water concerning industrial acid water or medical acid water.

### Background of the Invention

Patent Document 1 discloses the acid water invented by the present inventor where pH value is 2.6, oxidation-reduction potential is 450 mV, and chloride exists at 31 mg/L. This acid water is generated by "method for generating acid water and alkali water, an anode being placed in acid water generation area and a cathode in alkali water generation area, a voltage being applied to electrolyte which fills the acid water generation area and the alkali water generation area, respectively, generating acid water at the acid water generation area and alkali water at the alkali water generation area by ion exchange, wherein the electrolyte is generated by putting the nonmetallic mineral and the silicate mineral as electrolyzer into raw water and dissolving ionic material from the nonmetallic mineral and the silicate mineral in the raw water."

It has been derived from the inventor's various studies and experiments that the acid water as described above where the pH value and the oxidation-reduction potential and also the residual chlorine are little can be generated without hydrochloric acid or others being mixed, if its hydrogen ion concentration and sulfate ion concentration are increased.

However, it was impossible to lower the pH value of the acid water below 2.2, even if using the above-mentioned process. By further research, the inventor found that if a new process is added in the above process, it became possible to generate the acid water with lower pH value, lower oxidation-reduction potential and little residual chlorine.

Furthermore, as a result of the research and experiments with respect to use of the above mentioned acid water, the inventor found that the water can suitably be used particularly in a medical field such as medicine dentistry and pharmacy including a hospital and a dentist office.
Patent Document 1 Japanese Patent Publication (JPA) No. 2001-334271

### Disclosure of the Invention

The acid water generation device of the present invention is characterized by comprising:
- electrolytic cell where raw water is injected;
- diaphragm for electrolysis which compart the cell to an acid water generation area and an alkali water generation area;
- anode placed in the acid water generation area;
- cathode placed in the alkali water generation area;
- fossil earths put into the acid water generation area;
- voltage applying means for applying voltage to the anode and the cathode;
- filtering means for filtering acid water generated at the acid water generation area; and
- promoting means for promoting evaporation of oxygen dissolved in the acid water filtered by the filtering means.

In addition, the method for generating the acid water of the present invention is characterized by including the following steps:
- separating a cell where raw water is injected to an acid water generation area and an alkali water generation area;
- placing an anode of voltage applying means at the acid water generation area;
- placing a cathode of voltage applying means at the alkali water generation area;
- putting fossil earths into the acid water generation area;
- applying voltages to the anode and the cathode;
- filtering acid water generated at the acid water generation area; and
- vaporizing the filtered acid water.
   Furthermore, the acid water of the present invention is characterized in that the pH value is lower than 2.0, oxidation-reduction potential is 400 to 1000 mV and chlorine content is less than 0.05 mg/L.
   In addition, in the acid water including enhancer of the present invention, the reconstructing or regenerating enhancer of reconstructing or regenerating object is dissolved in the acid water generated by the acid water generation device or the above-mentioned acid water.
   In other words, the acid water including enhancer of the present invention preferably does not have bad influence against human body (e.g. little residual chlorine), and the enhancer preferably easily dissolves.
   In particular, if pH value of the acid water is equal to or lower than 2.6, bacteriocidation process of implant will become unnecessary by immersing the implant into the acid water due to its bacteriocidation effect.
   In addition, the reconstructive or a regenerative medical appliance of the present invention includes the implant immersed in the acid water with enhancer or the above-mentioned acid water.
   Furthermore, in the mouth wash liquid of the present invention, fluoroapatite or flavoring (mint or aero essence) is dissolved to acid water generated by the acid water generation device or the acid water.

### The best embodiment of the invention

Embodiments of the present invention will be explained with reference to drawings as follows.

### (Embodiment 1)

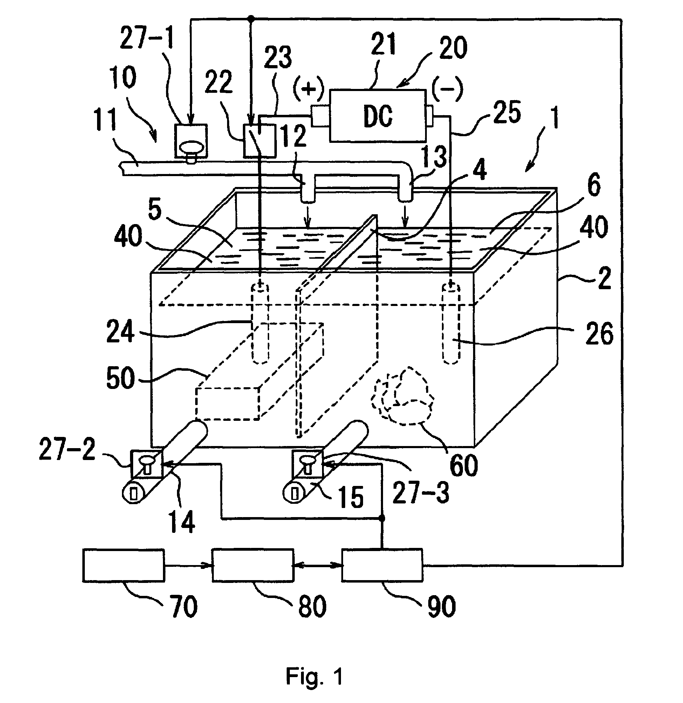

**FIG. 1** is a typical figure of constitution of an acid water producing device in embodiment 1 of the present invention. The producing device 1 comprises
- electrolytic cell 2 made of glass containers,
- water supply device 10 for supplying the electrolytic cell 2 with raw water,
- DC voltage applying device 20 for applying voltage to the raw water supplied by the water supply device 10,
- filtering means 70 for filtering acid water taken out from the electrolytic cell 2,
- promoting means 80 for promoting evaporation of water from acid water filtered by the filtering means 70, and
- controlling means for controlling the water supply device 10 side, DC voltage applying device 20 side and the promoting means 80 side.
   Partition wall 4 which has diaphragm for electrolysis 3 comprising nonwoven fabrics of polytetrafluoroethylene sections the inside of the electrolytic cell 2 into acid water generation area 5 and alkali water generation area 6.
   The water supply device 10 comprises a supply line 11 of tap water 40, which should be raw water, and an injection valve 27-1 of raw water is set in the supply line 11. In addition, the supply line 11 is respectively branched to one branch line 12 and another branch line 13. The opening of one branch line 12 faces the above of the acid water generation area and the opening of another branch line 13 faces the above of the alkali water generation area.
   An extract pipe 14 to extract acid water from the acid water generation area 5 and an extract pipe 15 to extract alkali water from generation area 6 is set on an electrolytic cell 2. Injection valves 27-2 and 27-3 of acid water and alkali water are set respectively at the extract pipe 14 and 15.
   In addition, a DC voltage applying device 20 comprises
- a DC voltage generator (battery) 21,
- a positive electrode pole (anode) 24 connected to the positive electrode side of the DC voltage generator 21 with a lead wire 23,
- negative electrode pole (cathode) 26 connected to the negative electrode side of the DC voltage generator 21 with the lead wire 23, and
- a switch 22 set on the lead wire 23.
   Furthermore, a positive electrode pole 24 is inserted in the acid water generation area 5 and a negative electrode pole 26 is inserted in the alkali water generation area 6, respectively.

The tap water 40 from the water supply device 10 is respectively supplied to the acid water generation area 5 and the alkali water generation area 6. A case 50 containing fossil earths, about 10% of the quantity of water, is put in to the acid water generation area 5. In addition, silicate mineral 60 such as tuff and zeolite is put in to the alkali water generation area 6.

For example, the tap water 40 should have more than 100 µS/cm electric conductivity, more than 20 mg/L mineral positive ion, more than 31 mg/L chloride ion, and higher than 50 degree hardness.

Fossil earths in the case 50 may be solidified sedimentary soil of algae fossil. In the present embodiment, solidified sedimentary soil of diatom fossil and solidified sedimentary soil of blue algae fossil are used. The case 50 containing fired fossil earths has plural number of through-pores.

Diatom earths is sedimentary soil of diatom shell, and diatom is a kind of algae floating in the sea and lake. In addition, diatom earth is a single celled plant, absorb nourishment from water and absorb silica to form cell membrane. Fossil of cell membrane is diatom shell. Fossil of this diatom shell forms independent cell of 0.1 micron - 10 micron, where the surface is covered with innumerable microscopic pores. This diatom earths, when existed in the earth, is siliceous fossil earths including material including hydrogen ion, sulfide and chloride by volcanic activity and crustal movement.

There are many kinds of blue algae such as oscillatoria and nostoc. They are single cells which can not move by themselves. In addition, blue algae live wandering or in massive or threaded colony, do not have any nucleus and reproduce only by fission. Blue algae fossilize, and when existed in the earth, it is siliceous fossil earths including hydrogen ion, sulfide and chloride by volcanic activity and crustal movement.

There are tens of kinds of natural fossil earths, and use of reddish-brown ones and ash black ones are preferable. Fossil earths comprise a lot of sedimentary layers, so when it is left untouched in water, water invades from innumerable microscopic pores on the surface, and water invades as soon as the space between sedimentary layers expands.

The fossil earth used here contains about 78% silicon (silicate), about 10 % aluminum (aluminum oxide), about 4.0 % iron (iron oxide), about 4.0 % potassium (potassium oxide), about 2.0 % calcium (calcium oxide), about 1.0 % sulfur (sulfate), and a small amount of nickel, strontium and rubinium.

The silicate mineral 60 used here contains about 78 % silicon (silicate), about 10 % aluminum oxide, about 4.0 % sodium oxide, about 4.0 % potassium oxide, about 1.0 % iron oxide, about 1.0 % magnesium oxide, about 1.0 % calcium (calcium oxide), and a small amount of sulfur, titanium, manganese, barium, zirconium, palladium and strontium. In addition, silicate mineral 60 is able to be replaced by sulfate mineral such as alunite or gypsum.

Filter paper less than 150 meshes is used in the filtering means 70. 100 meshes filter paper is preferable, and 50 meshes is more preferable. From the acid water filtered by the filtering means 70, impurities which were not ionized such as silicon and organic matters are removed.

Here, filtration process is employed for the following reason. That is, impurities remaining in the acid water are positive ions such as calcium, potassium and sodium. Therefore, if the following heating process is done without a filtration process, the impurities dissolve again due to the heating, and pH value of the acid water rise, as a result of which such pH value does not fall down.

As the promoting means 80, a heating means such as heater is used in the present embodiment. If heated higher than, for example, 60 °C, water evaporates from the acid water. In particular, it is preferable to be heated to higher than 78 °C to kill microorganism in acid water. Furthermore, when heated higher than the boiling point, vaporization of water is promoted, the hydrogen ion concentration and the sulfate ion concentration increase, and it will become possible to obtain the acid water where the pH value is lower than 2.0, oxidation-reduction potential is 400-1000 mV, and chloride content is less than 0.05 mg/L.

In addition, it was found that when vaporizing acid water and increasing the concentration 100-fold by heating, pH value reached 1.0. With respect to the acid water with pH value of 1.0, total hardness of is about 250 mg/L, specific electric conductivity is about 800 mS/m, oxidation-reduction potential is about 900 mV, and sulfate ion is about 1500 mg/L.

Controlling means 90 controls opening and closing of injection valve 27-1 of raw water, on/off of switch 22, extraction valve 27-2, 27-3 and drive of the promoting means 80. Controlling means 90 comprises memory input time data necessary for control and timer, and performs sequence control, or sets sensor on member of controlled object or member accompanying this and perform feedback control based on signal from sensor.

Then, generation process of acid water by acid water generation device 1 shown in FIG. 1 is explained. At first, fossil earths and silicate mineral 60 which are respectively put into the acid water generation area 5 and the alkali water generation area 6 prepare the electrolytic cell 2. Next, injection valve 27-1 of raw water is opened by controlling means 90. In this way, it injects raw water in the electrolytic cell 2. Next, after predetermined time has passed after opening injection valve 27-1, controlling means 90 closes injection valve 27-1. When the tap water 40 is injected into the electrolytic cell 2, ion materials elute from fossil earths and silicate mineral 60 to the tap water 40, and the tap water 40 becomes electrolytic solution (electrolyte).

It follows turning on switch 22 by controlling means 90 with DC voltage applying device 20 driven. In this way, voltage is applied to positive electrode pole 24 in the acid water generation area 5 and negative electrode pole 26 in the alkali water generation area 6. In addition, the relation between the tap water 40 and DC voltage by DC voltage applying device 20 is, about 5 mA to 10L tap water 40, about 50 mA to 100L tap water 40, about 0.5 A to 1 kL tap water 40, about 5 A to 10 kL tap water 40 and about 50 A to 100 kL tap water 40.

When electric current is passed to the tap water 40 which is electrolytic solution, among ion materials eluted from silicate mineral 60 in the alkali water generation area 6, negative ion material move to the acid water generation area 5 by selective permeation of diaphragm for electrolysis 3, and the hydrogen ion concentration of the acid water generation area 5 increases. In addition, among ion materials eluted from a nonmetallic mineral in the acid water generation area 5, positive ion material moves to the alkali water generation area side 6 by selective permeation of diaphragm for electrolysis 3. For this reason, the tap water 40 in the acid water generation area 5 and the tap water 40 in the alkali water generation area 6 are respectively reformed to acid water and alkali water.

Next, after predetermined time has passed after turning on switch 22, controlling means 90 turns off the switch. Then extraction valve 27-2 and 27-3 are opened. In this way, through extract pipe 14 and 15, acid water and alkali water are respectively extracted from the electrolytic cell 2. When enough time to finish extraction of acid water passes, extraction valve 27-2 and 27-3 are closed.

It follows acid water extracted from extract pipe 14 moved to the container which is not shown. Then, the filtering means 70 filters acid water in the container 70 and removes impurities and organic matters which were not ionized such as silicon. In addition, for above container, glass is the most preferable, secondly polyethylene, and thirdly polypropylene.

The acid water which filtered impurities enters the promoting means 80. Controlling means 90 controls heating temperature of acid water by the promoting means 80, for example, to be 98 degrees. In this way, vaporization of water is promoted, hydrogen ion concentration and sulfate ion concentration increase, and acid water where pH value is lower than 2.0, oxidation-reduction potential is 400 to 1000 mV, and chlorine content is less than 0.01 mg/L is able to be generated.

Here, when heating acid water, using container made of material that react little such as glass is preferable. Specifically heating in heat resisting glass is most preferable and heating in container made of ceramic is also preferable. Shape may be anything but shape such as conical flask is most preferable, secondly round shape flask, and thirdly volumetric flask.

In addition, acid water may be heated in a container made of stainless steel, and also in container made of enamel. In these cases, for example, a kettle may be used. In addition, also in these cases, shape such as conical flask is most preferable, secondly round shape flask, and thirdly volumetric flask.

Table 1 is a list showing the result of analysis of the acid water generated by the above process. This analysis of the acid water is report of examination and inspection performed by Japan Food Research Laboratories which the applicant requested.

**Table 1**

| Analysis Test Result | | | | |
|---|---|---|---|---|
| Analysis Test Item | Result | Detection Limit | Note | Method |
| pH | 1.9 (19°C) | | | glass electrode method |
| total hardness (CaCO₃) | 190 mg/L | | 1 | atomic absorption photometric method |
| electric conductivity (25°C) | 660 mS/m | | | electric conductivity meter method |
| oxidation-reduction potential | 690 mV | | | platinum electrode |
| (25°C vs. NHE) | | | | method |
| dissolved oxygen | 6.4 mg/L (20°C) | | | diaphragm electrode method |
| residual chlorine | not detected | 0.05 mg/L | | absorption photometric method |
| hydrogen peroxide | 0.22ppm | | | oxygen electrode method |
| chloride ion | 79 mg/l | | | ion chromatographic method |
| sulfate ion (SO₄²⁻) | 1,200 mg/L | | | ion chromatographic method |
| superoxide scavenging activity | not detected | 30 unit/g | 2 | electron spin resonance (ESR) method |
| organics and others | 6.2 mg/L | | 3 | |
| (potassium permanganate | | | | |
| consumption) | | | | |
| nitrate-nitrogen and | 4.2 mg/L | | | ion chromatographic |
| nitrite-nitrogen | | | | method |
| phosphorus | 0.02 mg/L | | | molybdenum blue absorption spectroscopy |
| copper | 4.4 mg/L | | | atomic absorption photometric method |
| zinc | 10 mg/L | | | atomic absorption photometric method |
| iron | 3.7mg/L | | | atomic absorption photometric method |
| manganese | 1.6 mg/L | | | atomic absorption photometric method |
| calcium | 45 mg/L | | | atomic absorption photometric method |
| magnesium | 19 mg/L | | | atomic absorption photometric method |
| sodium | 27 mg/L | | | atomic absorption photometric method |
| potassium | 5.0 mg/L | | | atomic absorption photometric method |

| | | | | |
|---|---|---|---|---|
| Note 1. Derived from measurements of calcium and magnesium Note 2. As scavenging ability equivalent to a unit [J. Biol. Chem., 244, 6049 (1969)] defined by J. M. McCord and I. Fridovich Note 3. Establishment of standards for tap water quality (Ministry of Health, Labour and Welfare, Notice No. 264, 1992) table 1 "inspection methods" | | | | |

As shown in table 1, pH value of acid water of the present embodiment is 1.9, oxidation-reduction potential is 690 mV and chlorine content (residual chlorine) is less than 0.05 mg/L. By the way, from this acid water, none of chloroform, bromodichloromethane and bromoform and trihalomethane was detected (respectively less than 0.0005 mg/L which is the detection limit). In addition, the result of analysis of this acid water is one example, and there is another that pH value reduced to 1.0 and there is another that oxidation-reduction potential is around 600 mV as stated already.

### Embodiment 2

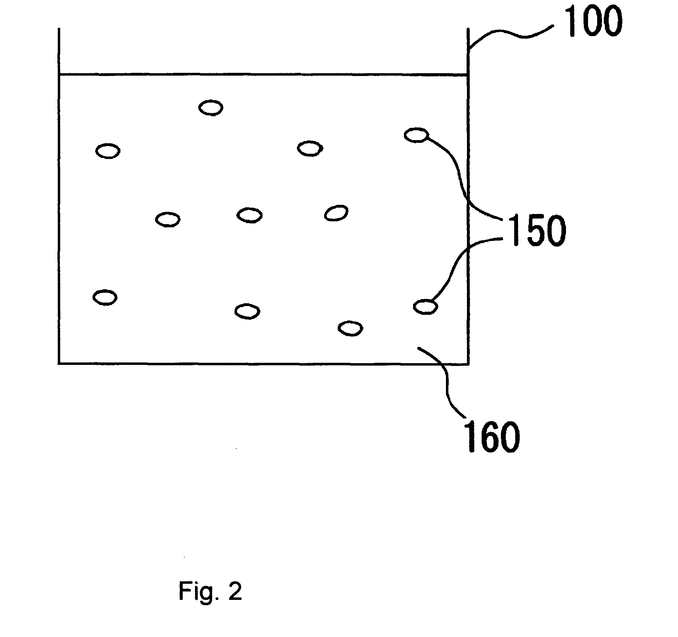

FIG. 2 is a typical figure of constitution of acid water including enhancer of embodiment 2 of the present invention. Container 100 shown in FIG. 2 is made of glass or plastic. For example, acid water 160 where the pH value is lower than 2.6, oxidation-reduction potential is 300 mV - 700 mV, chloride ion is less than 31 mg/L is injected into container 100

Less than saturated solubility of hydroxyapatite 150 which is enhancer is included in the acid water 160. For example, 800 mg of hydroxyapatite 150 is included in 1L of the acid water 160. In this case, calcium (Ca++) concentration of the acid water 160 is about 320 mg/L. This calcium concentration is about 3 times of the calcium concentration in body fluids.

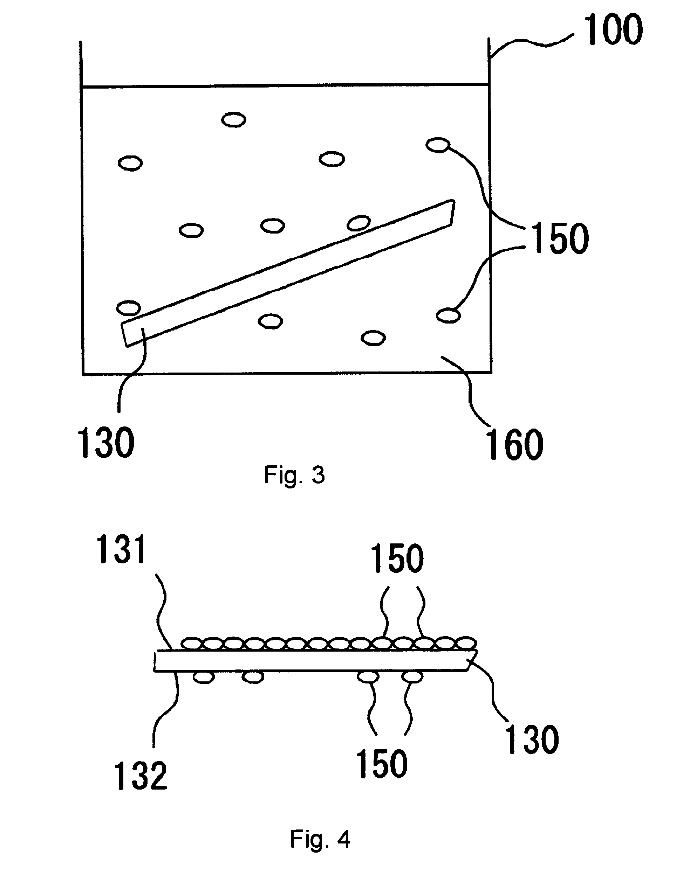

FIG.3 is a figure which shows membrane 130 immersed into container 100 where the acid water 160 shown in FIG. 2 is injected. One or both surfaces of Membrane 130 are plasma processed. In addition, in this case, the membrane 130 is made of polymeric material and plasma processing is effective to adhesion of hydroxyapatite 150 to the membrane 130.

When plasma processing is performed to the membrane 130, functional group including oxygen may be modified on the surface of the membrane 130. By this, wettability is improved and surface free energy increase. In addition, as surface of the membrane 130 is able to be roughed at the nano-level, above adhesion of hydroxyapatite 150 is effective. By the etching effect, adhesion of hydroxyapatite 150 becomes effective as discussed above.

In case of implants which use pure titanium and biodegradable polymer (polylactic acid) as materials, plasma processing is effective. However, when pure titanium is used as material, cleaning effect of the surface rather than adhesion of hydroxyapatite 150 is achieved by plasma processing.

Here, "implant" in present specification is "an alternative of organ and tissue artificially made to implant to the defected or injured area", including membrane, artificial bone, artificial organ, artificial skin, artificial nerve, artificial membrane, artificial joint or artificial tooth root.

FIG. 4 is a typical cross section view of the membrane 130 immersed in container 100 shown in FIG. 2, for example, for around 30 minutes. As shown in FIG. 4, relatively more hydroxyapatite 150 is adherent to surface 131 of the membrane 130 than the surface 132. Surface 131 is the surface that is plasma processed.

In fact, after performing plasma processing to half-masked surface 131 of the membrane 130, it is immersed in the acid water 160 with 800 mg/L hydroxyapatite 150 for around 30 minutes, for example. Then, observing the surface of the membrane 130 taken out from the acid water 160, hydroxyapatite 150 of 0.52 µg/cm² was adherent to not-masked area of surface 131 and hydroxyapatite 150 of 0.15 µg/cm2 was adherent to not-masked area of surface 132.

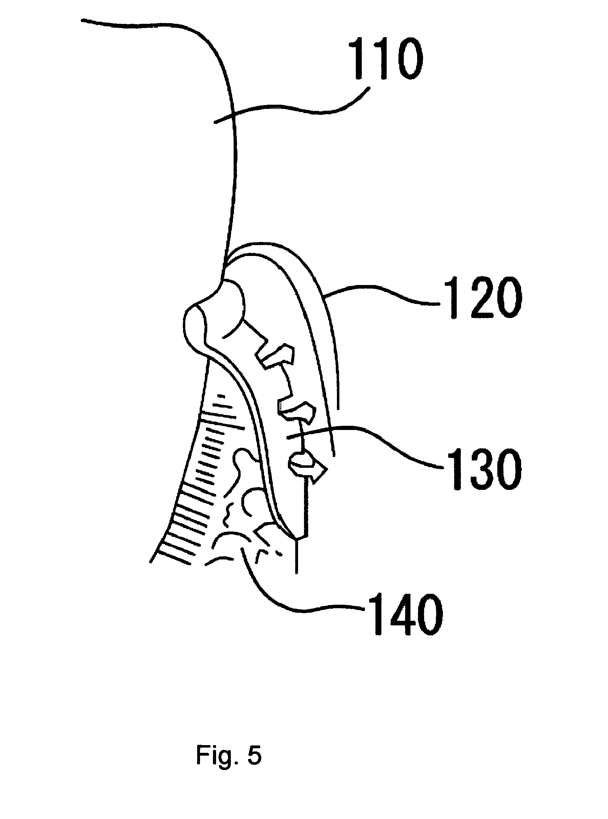

Fig. 5 is the illustration of application to treatment of periodontal disease of the membrane 130 where hydroxyapatite 150 is adherent shown in FIG. 2. At first, the area of periodontal disease is removed after cleaning area of tooth 110 and gingiva 120. Here, the membrane 130 is set so that supporting tissue 140 side is adhesion surface of hydroxyapatite 150. In this way, external invasion of another soft tissue under hydroxyapatite 150 is able to be prevented. Therefore, by original function of the membrane 130, fall of gingiva 120 was able to be prevented.

In this way, early treatment of the affected area is possible and reduction of patients' strain caused by treatment period is able to be improved. In addition, stated already, because adhesion of hydroxyapatite 150 to the membrane 130 is done in short time, there is a merit that adhesion process of hydroxyapatite 150 to the membrane 130 is able to be performed at the clinical field.

In addition, periimplantitis, inflammatory disease of tissue surrounding implant, also follow the condition similar to periodontal disease. These are diseases attracting attention among complications which lead to implant loss.

For treatment to periimplantitis, anti-inflammatory treatment, treatment of infrabony pocket and removal of implant are performed. For this treatment, asepticization of tainted implant surface and regenerative therapy is performed. Implant is generally said to be very difficult to asepticizate or detoxicate its surface for its surface property, but asepticizate of the surface was possible by immersing tainted implant into the acid water 160.

In addition, it was reported that, though mechanically grinding, or air-polishing or, using laser irradiation or acid, chemically cleaning the tainted implant, titanium surface once polluted was not able to be reosseointegrated, while reosseointegration was confirmed for the implant asepticizated by using the acid water 160. In addition, it is confirmed that the titanium oxide layer and ceramics are dissolved in the acid water 160. This point seems to contribute to reosseointegration.

Furthermore, because the acid water 160 where the pH value is lower than 2.6 is, as stated below, effective in bacteriocidation, there is a merit that bacteriocidation process of the membrane 130 taken out from the acid water 160 will be unnecessary. That is to say, when the membrane 130 is immersed into the acid water 160 including hydroxyapatite 150, bacteriocidation process of the membrane 130 and adhesion process of hydroxyapatite 150 to the membrane 130 will be able to be performed at a time, besides, at a short time. This point also contributes to possibility of adhesion process of hydroxyapatite 150 to the membrane 130 to be performed at the clinical field. In addition, the membrane 130 was able to be bacteriocided by acid water with pH value around 2.5 in around 1 hour, pH value around 1.5 in around 40 minutes and pH value around 1.0 in around 15 minutes. By the way, conventional representative method, bacteriocidation using ethylene oxide gas needed around 12 days.

When the membrane 130 precipitated by calcium phosphate was actually immersed into simulated body fluid (SBF), precipitating quantity of calcium phosphate increased. In this way, when the membrane 130 is embedded in organism, it is assumed that regenerating of osseous tissue will be earlier.

It was proved that dissolving quantity of hydroxyapatite 150 to the acid water 160 where the pH value is around 5.0 is also large. When using this acid water 160, sterilization procedure of the membrane 130 should be performed by gas sterilization.

In the present embodiment, use of the acid water 160 is stated taken hydroxyapatite as enhancer and implant as membrane for example, but enhancer, implant and these combinations are not limited to this. For example, enhancer only needs to be component of regenerating object including collagen, potassium apatite and other calcium phosphate or peptide. In addition, artificial bone, artificial organs, artificial skin, artificial nerves, artificial membrane, artificial joint or artificial tooth root is included in the implant.

In addition, the acid water 160 is able to be used as mouth wash liquid because it is effective in bacteriocidation. To be concrete, for example, at least either fluorine or flavorings need to be put into the acid water 160 to enhance recalcification of teeth. Combination of the acid water 160 and fluorine realize mouth bacteriocidation and recalcification of teeth, combination of the acid water 160 and flavorings realize mouth bacteriocidation and a refreshment, and combination of the acid water 160, fluorine and flavorings realize mouth sterilization, recalcification of teeth and refreshment.

Calcium of removed tooth immersed into the acid water 160 for about 5 minutes dissolved approximately 0.8 mg/l, about 1/37 as compared to solution of hydrochloric acid with the same pH value as the acid water 160. It is assumed that dissolving quantity from teeth decrease further because about 320 mg/l calcium is dissolved in the present mouth wash liquid.

Furthermore, it is able to be used for various bacteriocidation in hospital or dentist. Tweezers, metal appliance such as cutting or grinding tools, polymer appliance such as impression and complex appliance such as dental chair absorption system are all able to be bacteriocided. Therefore, expensive autoclave which only metal appliance is able to use need not be used for bacteriocidation. In addition, black light which holds safety issue need not be used for bacteriocidation. Besides, chemical which hold safety and waste issue need not be used for bacteriocidation.

In other words, the acid water 160 is safety, cheap and needs no waste disposal. Furthermore, because component or others of the acid water 160 do not change when heating, combination with autoclave will be enabled and bacteriocidation processing and high-bacteriocidation processing will be enabled in a short time.

In addition, when enhancer was put in the acid water 160 generated by method explained in embodiment 1, more enhancer was confirmed to dissolve in this acid water. When the membrane 130 was immersed into this acid water including enhancer for around 30 minutes, adhesion quantity of the enhancer to the membrane 130 largely increased, to near 1 µg/cm2. In addition, necessary time for bacteriocidation of the membrane 130 was decreased.

In addition, the acid water 160 is able to be used in various ways at medical field such as medicine, dentistry and pharmacy, not only at hospital or dentist. Practical use in regenerative medicine is expected in particular. To be concrete, reconstructing enhancer of bone is dissolved in the acid water 160. Immersing artificial bone there enables bacteriocidation of artificial bone and also adhesion of reconstructing enhancer.

Consequently, the reconstruction of a bone by the reconstructing enhancer adherent to the artificial bone is enhanced, said adhesion process and bacteriocidation process are able to be compiled in one process, early treatment is realized and bacteriocidation and reconstructing enhancer adhesion processing of artificial bone at medical field is enabled.

Furthermore, the acid water 160 is able to be used in processing of alternate aggregate. Now, most alternate aggregate is made of titanium. After embedding alternate aggregate into body, it is preferable when adhesion to surrounding cell is efficient, because recovery of said area is accelerated. When alternate aggregate made of titanium is actually put into acid water, surface dissolve by acid water affecting on titanium and irregularity at the nano-level is formed. After immersing alternate aggregate into the acid water 160 where the pH value is around 2.5 for about 15 minutes, it was able to be confirmed that surface irregularity was roughed about 2 times when the surface of alternate aggregate was observed by 400-power microscope. Furthermore, when immersed for three hours, it was able to confirm that surface irregularity was roughed to more than 3 times. By the formation of such irregularity, surface area of alternate aggregate increase, therefore, efficient adhesion of the cell is realized.

Furthermore, the acid water 160 is able to be applied to medicine for animal. For example, cow such as Holsteins may be infected with mastitis because staphylococcus aureus grow for use of tapper. In this case, it tends to be treated using expensive medicines which affect staphylococcus aureus, besides, only around 30% of the cows which suffered from mastitis recovered completely, so improvement of a treatment is necessary. Staphylococcus aureus was bacteriocided and mastitis was relieved when requirement of the acid water 160 was sprayed on affected part of a milk cow infected with mastitis every other day. Besides, it was discovered that around 70 % of the milk cow which suffered from mastitis recovered completely with the use of the acid water 160 where the pH value is around 2.5.

Furthermore, the acid water 160 is colorless, odorless and controls outbreak of phytoplankton and zooplankton. In addition, astringent effect is achieved. In addition, most of microbe and fungi perish by its sterilization, bacteriocidation, antibacteriation and insecticidation effect. For example, around 10 % acid water annihilates Escherichia coli and viable bacteria. In addition, it is able to be used as water bacteriocider for washing fresh vegetables.

In addition, the acid water 160 is able to be used as hot acid spring water and hot sulfate ion spring, bacteriocidation water for simple hot neutral springs (artificial hot spring) and water bacteriocider of swimming pools. Outbreak of parasite, maggot or others in filthy water is able to be controlled, and it is able to be used as sterilizer/ bacteriocider of wet towel, napkin, dining table, table and tableware in restaurant or hotel.

Furthermore, user of public facilities, public space, bus or train may carry paper napkin immersed in acid water for use of hygiene, cleaning or bacteriocidation. In addition, it is able to be used as a bacteriocidation and deodorant of various germs in public restrooms, insecticide of fairway and green at golf course and bacteriocidation of sandboxes at parks or others.

Furthermore, the acid water 160 is able to be used as plating preprocessing of metal such as iron, copper, zinc, brass and silver and degreasing process, oxide film removal, acid pickling or others of board and electrostatic removal which is preprocessing of semiconductor soldering process.

### Industrial Applicability

This acid water is able to be used as electrostatic removal acid water, insecticidal acid water, washing acid water, and antibacterial acid water.

## Claims

1. Acid water generation device **characterized by** comprising:
- electrolytic cell where raw water is injected;
- diaphragm for electrolysis which compart the cell to an acid water generation area and an alkali water generation area;
- anode placed in the acid water generation area;
- cathode placed in the alkali water generation area;
- fossil earths put into the acid water generation area;
- voltage applying means for applying voltage to the anode and the cathode;
- filtering means for filtering acid water generated at the acid water generation area; and
- promoting means for promoting evaporation of oxygen dissolved in the acid water filtered by the filtering means.

2. Acid water generation device according to claim 1 **characterized by** comprising controlling means for controlling opening and closing of injection valve of the raw water, on/off of switch to apply voltage to the anode and the cathode, opening and closing of extraction valve to extract acid water from the electrolytic cell and controlling means for controlling drive of promoting means.

3. Acid water generation device according to claim 1 or 2 **characterized in that** the filtering means use filter paper less than 150 meshes.

4. Acid water generation device according to any of Claim 1 to 3 **characterized in that** the promoting means is heating means for applying vaporization temperature of alkali component included in acid water generated in the acid water generation area.

5. Acid water generation device according to any of claim 1 to 4 **characterized by** comprising silicate mineral or sulfate mineral put into the alkali water generation area.

6. Method for generating acid water **characterized by** including the following steps:
comparting electrolytic cell where raw water is injected to the acid
water generation area and the alkali water generation area;
- placing anode of voltage applying means at the acid water generation area;
- placing cathode of voltage applying means at the alkali water generation area;
- putting fossil earth into the acid water generation area;
- applying voltage to the anode and the cathode;
- filtering acid water generated at the acid water generation area; and
- vaporizing the filtered acid water.

7. Acid water **characterized in that** pH value is lower than 2.0., oxidation-reduction potential is 400-1000 mV and chlorine content is less than 0.05 mg/L.

8. Acid water according to Claim 7 **characterized in that** the acid water is industrial acid water, hygienic management and washing medical appliance or medical acid water.

9. Acid water according to Claim 7 or 8 **characterized in that** the acid water is antistatic acid water, insecticidal acid water, washing acid water or antibacterial acid water.

10. Acid water including enhancer **characterized in that** reconstructing or regenerating enhancer of reconstructing or regenerating object is dissolved in acid water generated using acid water generation device according to any of Claim 1 to 5 or acid water according to any of Claim 7 to 9.

11. Acid water including enhancer according to Claim 10 **characterized in that** the enhancer is component of the object.

12. Acid water including enhancer according to Claim 10 or 11 **characterized in that** the enhancer is calcium phosphate, collagen or peptide.

13. Acid water including enhancer according to any of Claim 10 to 12 **characterized in that** the enhancer is included less than saturated solubility.

14. Acid water with implant **characterized in that** it includes acid water with electrolytic cell according to any of Claim 10 to 13 and implant immersed into the acid water.

15. Acid water with implant according to Claim 14 **characterized in that** the implant is membrane, artificial bone, artificial organ, artificial skin, artificial nerves, artificial membrane, artificial joint, or artificial tooth root.

16. Mouth wash liquid **characterized in that** fluoroapatite or flavoring is dissolved in acid water generated using acid water generation device according to any of Claim 1 to 5 or acid water according to any of Claim 7 to 9.
